(19) Europäisches Patentamt
European Patent Office
Office européen des brevets

(11) **EP 3 527 809 A1**

(12) **DEMANDE DE BREVET EUROPEEN**

(43) Date de publication:
**21.08.2019 Bulletin 2019/34**

(51) Int Cl.:
*F02D 41/14* (2006.01)
*G01N 27/00* (2006.01)
*G01N 21/00* (2006.01)
*F02D 41/28* (2006.01)
*F02D 41/26* (2006.01)
*G01M 15/10* (2006.01)
*F02D 41/00* (2006.01)

(21) Numéro de dépôt: **19156700.7**

(22) Date de dépôt: **12.02.2019**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**
Etats de validation désignés:
**KH MA MD TN**

(30) Priorité: **16.02.2018 FR 1851330**

(71) Demandeur: **IFP Energies nouvelles**
**92852 Rueil-Malmaison Cedex (FR)**

(72) Inventeurs:
• **FROBERT, Arnaud**
**92852 RUEIL-MALMAISON CEDEX (FR)**
• **CORDE, Gilles**
**92852 RUEIL-MALMAISON CEDEX (FR)**

(54) **SYSTEME EMBARQUE DE MESURE DES EMISSIONS POLLUANTES D'UN VEHICULE**

(57) La présente invention concerne un système embarqué de mesure des émissions polluantes (PSEE) d'un véhicule. Le système comporte au moins un capteur (CAP) placé en aval du système de post-traitement, et optionnellement un capteur branché sur la prise diagnostic du véhicule et des moyens informatiques (SIN) comportant des modèles (MOD VEH, MOD MOT, MOD POT). Selon l'invention, la détermination des émissions polluantes est basée sur le signal issu du capteur (CAP) et sur les modèles (MOD VEH, MOD MOT, MOD POT).

Figure 1

EP 3 527 809 A1

**Description**

La présente invention concerne le domaine des systèmes embarqués de mesure des émissions polluantes d'un véhicule.

Les émissions réelles des véhicules particuliers sont une source d'inquiétude pour le grand public, ainsi que pour le législateur. En effet, les dernières affaires (« Dieselgate » et ses développements) ont jeté un discrédit malvenu sur l'industrie automobile en démontrant que les constructeurs automobiles n'étaient pas honnêtes dans leur gestion des émissions polluantes. Il a été prouvé que des réglages différents étaient utilisés lors de l'homologation et en usage réel.

Pour répondre à ses inquiétudes et éviter d'autres scandales, la norme d'homologation a changé, en la rendant plus proche de la réalité (cycle d'homologation WLTC : « Worldwide harmonized Light vehicles Test Procédures » ou procédure d'essai mondiale harmonisée pour les voitures particulières et véhicules utilitaires légers) et en comparant systématiquement les émissions obtenues dans des conditions laboratoire et dans des conditions réelles (« Real Driving Emission » RDE). A cette fin, ont été développés des systèmes de mesure embarquables sur un véhicule particulier et / ou sur un véhicule de transport. Ces appareils, dénommés "PEMS" ou « Portable Emission Measurement Systems » sont coûteux, fragiles et nécessitent une maintenance importante car ils se doivent de respecter les critères de précision définis par la norme. Ils sont donc uniquement destinés à être utilisés par des spécialistes, soit lors du développement d'un véhicule, soit lors de son homologation.

En outre, il a été développé des méthodes de détermination des émissions polluantes au moyen de modèles et sans capteur. La demande de brevet FR 3049653 (WO17174239) décrit une telle méthode basée sur des modèles. Bien que donnant satisfaction en termes de rapidité, de facilité d'utilisation et de caractère représentatif (les modèles sont construits avec des paramètres du véhicule), le procédé décrit dans cette demande de brevet reste perfectible pour certains types de véhicules.

Pour pallier ces inconvénients, la présente invention concerne un système embarqué de mesure des émissions polluantes d'un véhicule. Le système comporte au moins un capteur placé en aval du système de post-traitement et optionnellement un capteur branché sur la prise diagnostic (OBD de l'anglais «On Board Diagnostic ») du véhicule et des moyens informatiques comportant des modèles. Selon l'invention, la détermination des émissions polluantes est basée sur le signal issu du capteur et sur les modèles. L'utilisation des modèles prenant en compte le signal d'au moins un capteur permet d'avoir une détermination précise et représentative des émissions polluantes. De plus, l'utilisation des modèles permet de limiter le nombre de capteurs, ce qui permet de limiter le coût et la maintenance du système embarqué de mesure, et assure une simplicité d'utilisation.

**Le système selon l'invention**

L'invention concerne un système embarqué de mesure des émissions polluantes d'un véhicule, ledit véhicule comportant un moteur à combustion interne et un système de post-traitement des gaz d'échappement dudit moteur, ledit système embarqué comportant au moins un capteur placé en aval dudit système de post-traitement dudit véhicule et un système informatique de détermination des émissions polluantes dudit véhicule au moyen de la mise en oeuvre d'un modèle du véhicule, d'un modèle de la motorisation dudit véhicule et d'un modèle de post-traitement dudit véhicule, ledit modèle de véhicule reliant ladite position et/ou l'altitude et/ou la vitesse dudit véhicule au couple et au régime dudit moteur, ledit modèle de motorisation reliant ledit couple et ledit régime dudit moteur aux émissions de polluants en sortie dudit moteur, et ledit modèle de post-traitement reliant lesdites émissions de polluants en sortie dudit moteur aux émissions de polluants en sortie dudit système de post-traitement. Lesdits modèles dudit système informatique sont configurés pour prendre en compte un signal issu dudit au moins un capteur afin de déterminer les émissions polluantes dudit véhicule.

Selon un mode de réalisation, ledit système informatique comprend un téléphone intelligent.

Avantageusement, ledit au moins capteur communique avec ledit téléphone intelligent au moyen d'une connexion sans fil.

De préférence, lesdits modèles dudit système informatique sont enregistrés dans un nuage informatique, ledit téléphone intelligent étant configuré pour échanger avec ledit nuage informatique.

De manière avantageuse, ledit téléphone intelligent mesure la position et/ou l'altitude et/ou la vitesse dudit véhicule pour déterminer les émissions polluantes au moyen desdits modèles.

Conformément à une mise en oeuvre, ledit système informatique comporte des moyens d'enregistrement des émissions polluantes déterminées au moyen desdits modèles.

Selon un aspect, ledit système embarqué de mesure comprend en outre un capteur branché sur la prise de diagnostic dudit véhicule.

Conformément à une caractéristique, ledit au moins un capteur est choisi parmi un capteur d'oxydes d'azote NOx, un capteur de monoxyde de carbone CO ou de dioxyde de carbone $CO_2$, un capteur de dioxygène $O_2$, un capteur

d'hydrocarbures non brûlés, un capteur de particules.

Avantageusement, ledit capteur d'oxyde d'azote NOx est choisi parmi capteur électrochimique, un capteur ultraviolet UV, un capteur infrarouge IR, un détecteur à chimie luminescente CLD.

De préférence, ledit capteur de monoxyde de carbone CO ou de dioxyde de carbone $CO_2$ est choisi parmi un capteur ultraviolet UV ou un capteur infrarouge IR.

Selon un aspect, ledit capteur de dioxygène $O_2$ est choisi parmi un capteur électrochimique, une mesure par diode LASER, un détecteur paramagnétique, un détecteur magnéto-pneumatique.

De manière avantageuse, ledit capteur de particules est choisi parmi un comptage par décharge CORONA, un opacimètre optique, un capteur de particules par accumulation, un comptage par vapeur de butanol, un comptage par irradiation des particules, un détecteur de fumée optique ou ionique, un détecteur d'atténuation beta BAM, un compteur de particules LASER.

De préférence, ledit système embarqué comporte un capteur d'oxydes d'azote NOx, et un capteur de dioxyde de carbone $CO_2$.

Conformément à un mode de réalisation, lesdits modèles sont configurés pour prendre en compte le signal issu dudit au moins un capteur par un calage desdits modèles par comparaison des émissions polluantes déterminées par lesdits modèles avec les émissions polluantes mesurées par ledit au moins un capteur.

Avantageusement, le calage est mis en oeuvre au moyen d'une méthode de résolution par les moindres carrés et/ou par un filtre de Kalman.

Selon une mise en oeuvre, ledit système informatique est configuré en outre pour déterminer un paramètre descriptif du trajet dudit véhicule.

Selon une caractéristique, lesdits modèles dépendent en outre d'au moins un paramètre macroscopique dudit véhicule.

De manière avantageuse, ledit système comporte des moyens d'acquisition dudit au moins un paramètre macroscopique depuis une base de données et/ou au moyen d'une interface avec un utilisateur.

## Présentation succincte des figures

D'autres caractéristiques et avantages du système selon l'invention, apparaîtront à la lecture de la description ci-après d'exemples non limitatifs de réalisations, en se référant aux figures annexées et décrites ci-après.

La figure 1 illustre le système embarqué de mesure selon un premier mode de réalisation de l'invention.
La figure 2 illustre le système embarqué de mesure selon un deuxième mode de réalisation de l'invention.

## Description détaillée de l'invention

La présente invention concerne un système embarqué de mesure des émissions de polluants d'un véhicule.

Le terme « polluants » désigne les oxydes d'azote (NOx), les particules, les monoxydes de carbone (CO), les dioxydes de carbone ($CO_2$), les hydrocarbures non brûlés (HC). Le système selon l'invention permet de déterminer les émissions d'au moins un, avantageusement plusieurs, et de préférence tous, de ces polluants.

Le véhicule comporte un moteur à combustion interne (ci-après désigné par le terme « moteur ») et un système de post-traitement des gaz d'échappement du moteur. Le moteur à combustion interne peut être un moteur essence ou un moteur Diesel. Le moteur peut réaliser à lui seul le déplacement du véhicule, ou peut faire partie d'un système de propulsion hybride. Le système de post-traitement permet de traiter les émissions de polluants en sortie du moteur, diminuant ainsi les émissions de polluants du véhicule. Le système de post-traitement peut comporter un catalyseur trois voies pour traiter simultanément les hydrocarbures imbrûlés, le monoxyde de carbone et les oxydes d'azote et/ou un catalyseur d'oxydation pour traiter les hydrocarbures imbrûlés et le monoxyde de carbone, et/ou des catalyseurs DeNOx pour réduire les oxydes d'azote en présence d'oxygène, et/ou différents filtres pour éliminer les particules solides.

Le terme « embarqué » indique que le système est monté à bord du véhicule, ce qui inclut également la possibilité d'attacher le système à l'extérieur du véhicule.

Le système embarqué de mesure peut être utilisé pour les véhicules automobiles. Toutefois, il peut être utilisé dans le domaine du transport routier, le domaine des deux-roues, le domaine ferroviaire, le domaine naval, le domaine aéronautique, le domaine des aéroglisseurs, et le domaine des véhicules amphibies, etc.

Le système embarqué de mesure selon l'invention comporte :

- au moins un capteur placé en sortie du système de post-traitement, le capteur est prévu pour mesurer la quantité d'un gaz ou de particules en sortie du système de post-traitement,
- un système informatique pour déterminer les émissions polluantes, dans ce but, le système informatique exécute trois modèles en cascade (en série) :

◦ un modèle du véhicule, le modèle de véhicule relie la position et/ou l'altitude et/ou la vitesse du véhicule au couple et au régime du moteur,

◦ un modèle de motorisation du véhicule, le modèle de motorisation relie le couple et le régime du moteur aux émissions de polluants en sortie du moteur, et

◦ un modèle de post-traitement, le modèle de post-traitement relie les émissions de polluants en sortie du moteur aux émissions de polluants en sortie du système de post-traitement.

Selon l'invention, les trois modèles du système informatique sont configurés pour prendre un compte un signal issu du ou des capteur(s) afin de déterminer les émissions polluantes du véhicule et d'avoir des modèles recalés avec les mesures.

L'utilisation des modèles et d'au moins un capteur permet d'avoir une détermination précise et représentative des émissions polluantes, en effet les mesures issues du capteur permettent une calibration précise des modèles. De plus, l'utilisation des modèles permet de limiter le nombre de capteurs, impliquant une réduction du coût et une réduction des phases de maintenance du système de mesure, et de rendre le système facilement transportable et adaptable à tous types de véhicule.

L'obtention de modèles recalés avec les mesures par le(s) capteur(s) du système selon l'invention permet une exécution des modèles dans d'autres conditions et/ou avec d'autres profils de conduite, sans capteur, tout en présentant des résultats précis.

Optionnellement, le système embarqué de mesure peut comprendre un capteur qui se branche sur la prise de diagnostic du véhicule (OBD - « On board diagnostic ») et qui peut récupérer des valeurs comme le régime, la température moteur, la température de l'eau ou encore la vitesse véhicule. Dans la suite de la description, ce capteur est nommé capteur OBD. Les moyens informatiques peuvent être alors prévus pour prendre en compte les valeurs obtenues par le capteur OBD. Ces paramètres servent à recaler les modèles.

Selon un mode de réalisation de l'invention, le système informatique du système embarqué de mesure peut comprendre un téléphone intelligent (« smartphone »). Un tel téléphone intelligent permet une facilité d'installation dans le véhicule, une adaptabilité, une transportabilité, et une réduction des coûts par rapport à un ordinateur ou un calculateur dédié. De plus, l'utilisation d'un téléphone intelligent permet une utilisation du système par un large public.

Afin de faciliter le montage à bord du véhicule (absence de câblage spécifique), le ou les capteur(s) peut(peuvent) communiquer avec le téléphone intelligent au moins d'une connexion sans fil, par exemple du type wifi ou Bluetooth. Le cas échéant, le capteur OBD peut également communiquer avec le téléphone intelligent au moyen d'une connexion sans fil. En variante, une connexion filaire peut être prévue entre le capteur et le téléphone intelligent ou un ordinateur ou un calculateur.

En outre, les trois modèles du système informatique peuvent être enregistrés dans un nuage informatique (« cloud ») ou un service en ligne. Cela permet de limiter la mémoire consommée sur le téléphone intelligent, et d'utiliser des processeurs plus puissants pour exécuter les trois modèles. Pour cette configuration, le téléphone intelligent est configuré pour échanger avec le nuage informatique au moyen d'une connexion internet. Cette configuration utilisant à la fois un téléphone intelligent et un nuage informatique permet une utilisation simplifiée à coût réduit.

Si le téléphone intelligent est équipé avec un système de géolocalisation (GPS), alors le téléphone intelligent peut être configuré en outre pour mesurer la vitesse et/ou l'altitude et/ou la position du véhicule. Ces données de position, vitesse ou altitude peuvent être utilisées dans les modèles du système informatique, en particulier dans le modèle du véhicule.

En variante (par exemple si le téléphone est dépourvu d'un système de géolocalisation), le système embarqué peut comprendre un capteur de géolocalisation qui communique avec le téléphone intelligent.

Selon un autre aspect, le système informatique peut comprendre en outre un moyen d'affichage des émissions de polluants déterminées au moyen des modèles, afin d'informer l'utilisateur du véhicule des émissions polluantes. Par exemple, le moyen d'affichage peut être l'écran d'un téléphone intelligent.

Alternativement au téléphone intelligent, le système informatique peut comprendre un calculateur ou un ordinateur qui communique avec le ou les capteur(s). Ce calculateur ou ordinateur peut intégrer les trois modèles.

Selon un aspect de l'invention, les moyens informatiques peuvent comprendre des moyens de stockage (d'enregistrement) des valeurs des émissions polluantes déterminées par le système embarqué de mesure. Les moyens de stockage peuvent permettre également de stocker les modèles recalés avec les mesures.

Par exemple, ces moyens de stockage peuvent être inclus dans le téléphone intelligent et/ou dans le nuage informatique.

Conformément à une mise en oeuvre de l'invention, le capteur peut être choisi parmi un capteur OBD, un capteur d'oxydes d'azote NOx, un capteur de monoxyde de carbone CO ou de dioxyde de carbone $CO_2$, un capteur de dioxygène $O_2$, un capteur d'hydrocarbures non brûlés, ou un capteur de particules.

De préférence, le système embarqué de mesure comporte un capteur de dioxyde de carbone $CO_2$, et un capteur de d'oxydes d'azote NOx. Cette configuration permet un bon compromis en termes de coût du système embarqué, et de données mesurées pour une calibration optimale des modèles.

En particulier, le ou les capteur(s) du système embarqué de mesure peuvent être choisis parmi :

○ en ce qui concerne le capteur OBD :

- Préférentiellement, un capteur capable de lire les informations circulant sur le réseau de communication «OBD» ou On Board Diagnostic du véhicule, par l'intermédiaire de la prise diagnostic ou prise « OBD », située à proximité du tableau de bord du véhicule.

○ en ce qui concerne les capteurs d'oxydes d'azote NOx volumiques :

- Préférentiellement, un capteur électrochimique,
- un capteur ultraviolet UV,
- un capteur infrarouge IR, ou
- un détecteur à Chimie-Luminescence (CLD),

○ en ce qui concerne les capteurs de monoxyde de carbone ou de dioxyde de carbone $CO/CO_2$ volumique :

- Préférentiellement, un capteur infrarouge IR, ou
- un capteur ultraviolet UV,

○ en ce qui concerne les capteurs de dioxygène $O_2$ volumique :

- Préférentiellement, un capteur électrochimique,
- une mesure par diode laser,
- un détecteur Paramagnétique, ou
- un détecteur Magnéto-Pneumatique,

○ en ce qui concerne les capteurs de particules PM/PN/Opacité :

- Préférentiellement, un capteur de particules par accumulation,
- un comptage par décharge CORONA,
- un opacimètre optique,
- un comptage par vapeur de butanol,
- un comptage par irradiation des particules,
- un détecteur de fumée optique ou ionique,
- un détecteur d'atténuation béta BAM (« Beta Attenuation Monitors ») qui mesure la qualité de l'air, ou
- un compteur de particules laser (True Laser Particle Counter) qui mesure la qualité de l'air.

Pour la prise en compte des signaux issus du ou des capteur(s) dans les modèles, le système informatique peut être configuré pour réaliser un apprentissage en continu des modèles. Selon un exemple, cet apprentissage en continu peut consister en un calage des modèles. Il peut s'agir d'un calage par comparaison des émissions polluantes déterminées par les modèles avec les émissions polluantes mesurées par le ou les capteurs. Par exemple, si le capteur est un capteur de dioxyde d'azote NOx, alors la valeur mesurée peut être comparée à la valeur de dioxyde d'azote déterminée par les modèles. Comme exemples de recalage possible, on peut citer :

- Un recalage des rapports de transmission du véhicule grâce à une valeur de régime moteur,
- Un recalage de la dynamique de montée en température du moteur grâce à une température d'eau moteur,
- Un recalage de la consommation de carburant grâce à la valeur de dioxyde de carbone $CO_2$,
- Dans le cas d'une motorisation diesel, les valeurs mesurées de dioxyde de carbone $CO_2$ et d'oxydes d'azote NOx permettent avantageusement de recaler la cartographie de recirculation des gaz brûlés d'un système de recirculation des gaz brulés EGR (cartographie BGR) issu d'un modèle,
- La valeur de monoxyde de carbone CO permet de recaler les modèles d'amorçage / de mise en température des catalyseurs trois voies ou d'oxydation,
- Dans le cas d'un moteur à allumage commandé (par exemple à carburant essence), la valeur d'oxydes d'azote NOx permet de recaler l'amorçage / la mise en température du catalyseur trois voies,
- La valeur d'oxydes d'azote NOx permet de recaler les modèles liées aux catalyseurs de réduction des NOx : modèle de stockage NOx dans le cas d'un piège à NOx, modèle d'injection et de stockage de réducteur (le plus souvent ammoniac) dans le cas d'un catalyseur RCS (Réduction catalytique sélective ou SCR),

- La valeur de particules mesurée permet de recaler les modèles d'émissions de suies quand le moteur ne dispose pas de système de filtration des gaz d'échappement,
- Si le moteur dispose d'un système de filtration des gaz d'échappement, la mesure de particules permet de juger de l'efficacité de filtration.

Dans tous les cas cités précédemment, le fait d'avoir une mesure permet avantageusement de recaler les modèles phénoménologiques mais aussi de mettre en évidence les véhicules défaillants ou modifiés qui s'éloignent des standards.

Selon un aspect de l'invention, les moyens informatiques peuvent être configurés pour réaliser le calage au moyen d'une méthode des moindres carrés et/ou au moyen d'un filtre de Kalman. Dans les deux cas, les signaux obtenus via le modèle et les signaux physiques sont comparés et ensuite, les paramètres du modèles sont modifiés pour aligner les sorties des modèles avec les valeurs physiques obtenues par les capteurs.

Selon un mode de réalisation de l'invention, les trois modèles du système informatiques peuvent être dépendants de paramètres macroscopiques du véhicule. Ainsi, les modèles représentent au mieux le véhicule, la motorisation et le système de post-traitement. On appelle paramètre macroscopique, une caractéristique générale relative au véhicule, son moteur ou son système de post-traitement. Il s'agit de paramètre constant pour un véhicule, correspondant à des données du constructeur du véhicule. Le paramètre est dit macroscopique car il est déterminé à l'échelle du véhicule, et il ne s'agit pas de paramètre microscopique qui peut être déterminé, comme par exemple dans la demande de brevet FR2984557 (US 2013/0158967), à l'échelle d'une maille représentant une petite portion de la chambre de combustion.

Les paramètres macroscopiques peuvent être de trois types :

- des paramètres liés à construction générale du véhicule (par exemple : masse du véhicule, transmission, ...)
- des paramètres liés à la motorisation (par exemple : le type d'injection, la cylindrée, le type de motorisation...), et
- des paramètres liés au système de post-traitement (par exemple : type de post-traitement).

Selon un mode de réalisation de l'invention, on peut acquérir au moins un paramètre macroscopique choisi parmi :

- le type de motorisation (essence, Diesel...),
- le niveau de norme d'homologation (Euro 1, Euro 2, ...),
- la cylindrée du moteur,
- le couple maximal et le régime du moteur associé,
- la puissance maximale et le régime du moteur associé,
- la masse du véhicule,
- le type de transmission du véhicule (type et constitution de boîte de vitesse,...),
- le type de système de post-traitement,
- le type du système d'injection,
- l'architecture de la boucle d'air (présence/absence d'une recirculation des gaz brûlés notée EGR, utilisation d'un turbocompresseur, d'une suralimentation...),
- les dimensions des roues, etc.

Selon une variante de réalisation, les paramètres macroscopiques peuvent être obtenus depuis une base de données, qui répertorie les différents véhicules en circulation. Par exemple, les paramètres macroscopiques peuvent être obtenus en indiquant le numéro d'immatriculation du véhicule, la base de données associant le numéro de plaque à sa conception (marque, modèle, motorisation...), et comprenant les paramètres macroscopiques du véhicule. Selon un exemple de réalisation, la base de données peut être enregistrée dans le nuage informatique du système informatique.

Alternativement, les paramètres macroscopiques peuvent être des données constructeurs renseignés par l'utilisateur, en particulier au moyen d'une interface (par exemple un téléphone intelligent ou un système de géolocalisation).

Conformément à une mise en oeuvre de l'invention, le système embarqué de mesure peut être configuré, au moyen du système informatique, pour déterminer au moins un paramètre descriptif d'un trajet parcouru par le véhicule. Par exemple, le paramètre descriptif peut être le type de trajet (urbain, extra-urbain, etc.), le type de conduite (agressif, médian, souple), le dénivelé, la circulation, la conformité aux besoins RDE (« Real Driving Emission » mesure d'émissions en conduite réelle). Ainsi, il est possible d'associer à chaque trajet, un paramètre le caractérisant, et les émissions de polluants du véhicule.

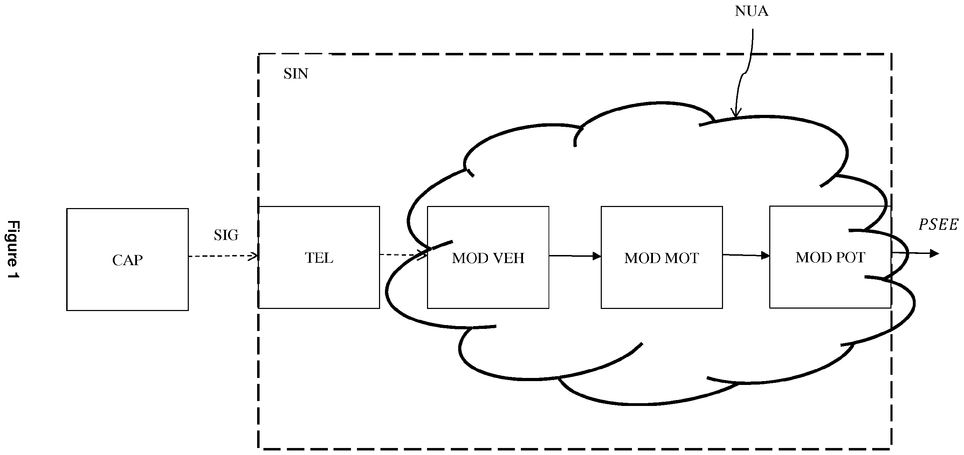

La figure 1 illustre, schématiquement et de manière non limitative, un système embarqué de mesure selon un mode de réalisation de l'invention. Le système embarqué de mesure comporte un capteur CAP agencé en sortie du système de post-traitement du véhicule (non représenté). Le système embarqué de mesure comporte un système informatique SIN. Le système informatique SIN comporte un téléphone intelligent TEL et un nuage informatique NUA.

Sur cette figure, les flèches en traits pointillés correspondent à des connexion sans fil entre les éléments. Le capteur CAP transmet un signal SIG issu de la mesure du capteur au téléphone intelligent TEL par une connexion sans fil, par exemple Bluetooth. De plus, le téléphone intelligent TEL échange avec le nuage informatique NUA par une connexion sans fil, par exemple une connexion GSM (« Global System for Mobile Communications », système global de communications pour les téléphones portables) . Le téléphone intelligent transmet le signal SIG issu du capteur. En outre, le téléphone intelligent peut transmettre une mesure de géolocalisation.

Au sein du nuage informatique NUA sont stockées trois modèles : un modèle de véhicule MOD VEH, un modèle de motorisation MOD MOT, et un modèle de post-traitement MOD POT. Le système informatique SIN exécute ces trois modèles les uns après les autres au moyen des données reçues par le téléphone intelligent, pour déterminer les émissions de polluants PSEE. Selon l'invention, le signal SIG du capteur CAP est utilisé pour calibrer au moins un des trois modèles.

Dans la suite de la description, on décrit, de manière non limitative, des exemples de modèles du système informatique du système embarqué de mesure selon l'invention.

Notations

Dans la suite de la description, on utilise les notations suivantes :

| | | |
|---|---|---|
| $pos_{GPS}$ | Coordonnées mesurées par géolocalisation dans le repère de Lambert | [m] |
| $alt_{GPS}$ | Altitude mesurée par géolocalisation | [m] |
| $v_{GPS}$ | Vitesse du véhicule mesurée par géolocalisation | [m/s] |
| v | Vitesse du véhicule | [m/s] |
| Ne | Régime du moteur estimé | [tr/min] |
| Cme | Couple du moteur estimé | [Nm] |
| m | Masse du véhicule | [kg] |
| $F_T$ | Effort de traction du véhicule à la roue | [N] |
| $F_{res}$ | Résultante des efforts de frottement subis par le véhicule | [N] |
| $F_{slope}$ | Effort normal subi par le véhicule (gravité) | [N] |
| $F_{brk}$ | Force de freinage mécanique | [N] |
| $\alpha$ | Angle d'inclinaison de la route | [rad] |
| a, b, c | Paramètres du véhicule | [-] |
| Pe | Puissance moteur estimée | [kW] |
| $\eta_{trans}$ | Rendement de la transmission | [-] |
| $R_{MTH\text{-}v}$ | Rapport de réduction entre le régime de rotation du moteur et la vitesse du véhicule | [rpm/km/h] |
| PSME | Emissions polluantes en sortie du moteur | [g/s] |
| PSME, | Emissions du polluant i en sortie du moteur | [g/s] |
| $PSMEi_{\text{-}QS}$ | Emissions du polluant i en sortie du moteur pour un régime quasi-statique | [g/h] |
| $NOx_{QS}$ | Masse de NOx par unité de masse de carburant | [g/kg de carburant] |
| COC | Angle de vilebrequin au centre de combustion (50% de conversion d'énergie) | [°CA] |
| $m_{cyl}$ | Masse d'air enfermée dans le cylindre par cycle | [g/$10^3$cm$^3$] |
| $m_{O2}$ | Masse d'oxygène enfermée dans le cylindre par cycle | [g/$10^3$cm$^3$] |
| $a_0, a_1, a_2, a_3$ | Coefficients | [-] |
| $Soot_{QS}$ | Emissions de particules en sortie du moteur en régime quasi-statique | [g/s] |
| BGR | Fraction de gaz brûlés dans le cylindre | [%] |
| $BGR_{dyn}$ | Fraction dynamique de gaz brûlés dans le cylindre | [%] |
| $AF_{ratio}$ | Richesse du mélange dans le cylindre | [-] |
| $AF_{ratio\text{-}dyn}$ | Richesse dynamique du mélange dans le cylindre | [-] |
| $Cor_{i\text{-}QS2TR}$ | Coefficient de correction pour l'impact des phénomènes transitoires pour le polluant i | [-] |
| PSEE | Emissions polluantes en sortie du système de post-traitement | [g/s] |

(suite)

| | | |
|---|---|---|
| PSEE$_i$ | Emissions du polluant i en sortie du système de post-traitement | [g/s] |
| COnv$_{i,j}$ | Efficacité de conversion de la tranche j du système de post-traitement pour le polluant i | [-] |
| Téch | Température des gaz d'échappement | [K] |
| Qéch | Débit des gaz d'échappement | [g/s] |

Pour ces notations, la dérivée par rapport au temps est notée $\frac{d}{dt}$.

Dans la présente demande, le terme f désigne de manière générale une fonction, qui peut être de tout type.

Selon l'invention, on construit trois modèles macroscopiques afin de déterminer les émissions de polluants. Ces modèles sont de préférence paramétrés avec les paramètres macroscopiques acquis, permettant ainsi de rendre la détermination des émissions polluantes représentative du véhicule. Il s'agit d'un modèle du véhicule, d'un modèle du moteur et d'un modèle de post-traitement (c'est-à-dire un modèle du système de post-traitement).

Modèle de véhicule

Le modèle du véhicule relie la position et/ou l'altitude et/ou la vitesse du véhicule au couple et au régime du moteur, de préférence au moyen d'au moins un paramètre macroscopique. Selon une mise en oeuvre de l'invention, pour construire le modèle du véhicule on peut utiliser au moins un des paramètres macroscopiques suivants : masse du véhicule, puissance maximale et le régime du moteur associé, vitesse maximale, type de transmission....

Selon un mode de réalisation de l'invention, on peut construire le modèle du véhicule par association d'un modèle de dynamique du véhicule et d'un modèle de transmission du véhicule. Le modèle de dynamique du véhicule relie la position et/ou la vitesse et/ou l'altitude du véhicule à la puissance estimée du véhicule au moyen d'au moins un paramètre macroscopique, par exemple la masse du véhicule, le type de transmission, les dimensions des roues. Le modèle de transmission du véhicule relie la puissance du véhicule au régime et au couple du moteur, au moyen d'au moins un paramètre macroscopique, par exemple le type de transmission, la puissance maximale et le régime moteur associé.

Le modèle de dynamique du véhicule prend en compte la dynamique du véhicule. Il peut être construit à partir de l'application du principe fondamental de la dynamique du véhicule appliqué sur son axe longitudinal, et peut s'écrire sous la forme suivante :

$$m\frac{dv}{dt} = F_T - F_{res} - F_{slope} - F_{brk}$$

avec :

v est la vitesse du véhicule, une entrée du modèle de la dynamique du véhicule.

$F_{res}$ peut être exprimée en fonction de la vitesse sous la forme $F_{res} = a + bv + cv^2$ avec a, b, c des paramètres du véhicule à identifier en fonction des caractéristiques générales du véhicule (paramètres macroscopiques du véhicule).

$F_{slope}$ peut être exprimée en fonction de la masse du véhicule et de l'inclinaison $\alpha$ de la route : $F_{slope} = mg\sin(\alpha)$. L'angle d'inclinaison $\alpha$ est une donnée d'entrée du modèle de la dynamique du véhicule. En effet, l'inclinaison $\alpha$ peut être calculée à partir de l'altitude et de la distance parcourue, elle dépend donc de l'altitude et de la position. Selon un mode de réalisation, l'angle d'inclinaison $\alpha$ peut être déterminé par une formule du type :

$$\alpha = \arctan\left(\frac{\Delta altitude}{\Delta distance}\right)$$

Ces équations permettent d'écrire une formule qui relie la puissance estimée du moteur à la vitesse du véhicule et d'autres paramètres macroscopiques connus ou déterminables. En effet, on peut écrire l'équation :

$$Pe = F_T * v/\eta_{trans}$$

Ainsi, en combinant les différentes équations, il est possible de déterminer une formule qui relie la puissance du moteur à la vitesse et l'altitude du véhicule, au moyen de paramètres macroscopiques connus et constants.

Le modèle de transmission estime le rapport de réduction entre le régime de rotation du moteur thermique et la vitesse du véhicule. Il peut être paramétré en fonction des caractéristiques générales (paramètres macroscopiques) du véhicule, notamment la masse du véhicule, la puissance maximale, le type de transmission, en particulier le nombre de rapports. Ce modèle de transmission utilise seulement la vitesse du véhicule en tant que donnée d'entrée, pour estimer le rapport de réduction :

$$R_{MTH-v} = f(v)$$

La fonction f peut être obtenue notamment à partir d'abaques données par le constructeur et peut être calée en cours de fonctionnement au moyen des mesures d'un capteur. Par exemple, le cas échéant, la fonction f peut être recalée avec un capteur branché sur la prise de diagnostic du véhicule OBD.

Ce rapport de réduction peut ensuite être utilisé pour déterminer le régime du moteur. En effet, on peut écrire les relations suivantes :

$$Ne = R_{MTH-v} * v$$

Ensuite, le couple du moteur peut être déterminé en fonction de la puissance (estimée à l'aide du modèle de dynamique du véhicule) et du régime du moteur :

$$Cme = f(Ne, Pe)$$

La fonction f peut être obtenue par des cartographies données par le constructeur et peut être calée en cours de fonctionnement au moyen des mesures d'un capteur.

Modèle du moteur

Le modèle du moteur relie le régime et le couple du moteur aux émissions de polluants en sortie du moteur (c'est-à-dire avant le système de post-traitement), de préférence au moyen d'au moins un paramètre macroscopique. Selon une mise en oeuvre de l'invention, pour construire le modèle du moteur, on peut utiliser au moins un des paramètres macroscopiques suivants : la cylindrée, le type de motorisation, le couple et la puissance, l'architecture de la boucle d'air, la norme d'homologation du véhicule, etc.

Selon un mode de réalisation de l'invention, on peut construire le modèle du moteur par association d'un modèle énergétique et d'un modèle de polluants en sortie du moteur. Le modèle énergétique relie le couple et le régime du moteur à des débits et des températures de fluides mis en oeuvre dans le moteur à combustion (carburants, gaz à l'admission, gaz à l'échappement, éventuellement recirculation des gaz brûlés) de préférence au moyen d'au moins un paramètre macroscopique, par exemple la cylindrée, le type de motorisation, le couple et la puissance maximaux, l'architecture de la boucle d'air. Le modèle de polluants en sortie moteur relie des débits et des températures de fluides mis en oeuvre dans le moteur à combustion interne aux émissions de polluants en sortie du moteur, au moyen d'au moins un paramètre macroscopique, par exemple la norme d'homologation du véhicule, le type de motorisation, l'architecture de la boucle d'air.

Le modèle énergétique permet d'estimer les grandeurs physiques sur le point de fonctionnement courant (régime, couple). Il est paramétré en fonction de paramètres macroscopiques. Les grandeurs physiques estimées sont les débits et températures des fluides mis en oeuvre dans le moteur à combustion (carburants, gaz à l'admission, gaz à l'échappement, éventuellement recirculation des gaz brûlés).

Le modèle de polluants en sortie du moteur permet à partir de l'information du régime et du couple du moteur, et des estimations issues du modèle énergétique, d'estimer les émissions polluantes en sortie du moteur. Il peut être paramétré en fonction des caractéristiques générales du véhicule et du moteur : la norme d'homologation du véhicule, le type de motorisation, l'architecture de la boucle d'air, etc.

L'estimation des polluants en sortie moteur peut se faire en deux étapes :

- estimation des émissions en quasi-statique au moyen d'un modèle quasi-statique, et
- estimation de l'impact des phénomènes transitoires au moyen d'un modèle transitoire.

Alternativement, l'estimation des polluants en sortie moteur peut se faire uniquement en une seule étape au moyen du modèle quasi-statique.

Estimer les émissions en quasi-statique d'un moteur sur un point de fonctionnement à un instant donné revient à considérer que ce moteur est en fonctionnement stabilisé sur ce point de fonctionnement.

L'estimation de l'impact des phénomènes transitoires (fonctionnement non stabilisé) permet de prendre en compte les phénomènes transitoires, qui engendrent généralement un surplus d'émissions polluantes.

Les modèles quasi-statiques de polluants peuvent être paramétrés au moyen de paramètres macroscopiques du véhicule et du moteur. Ils permettent à chaque instant d'estimer les émissions polluantes quasi-statiques en sortie moteur, à partir des estimations de régime et de couple du moteur thermique et des sorties du modèle énergétique. Les modèles quasi-statiques peuvent s'écrire sous la forme :

$$PSME_{i-QS} = f(Ne, Cme)$$

La fonction f peut être de différent type, en fonction du type de polluant étudié et peut être calée en cours de fonctionnement au moyen des mesures d'un capteur.

Par exemple, le modèle quasi-statique de NOx peut être issu des travaux de Gartner, (U. Gartner, G. Hohenberg, H. Daudel and H. Oelschlegel, Development and Application of a Semi-Empirical NOx Model to Various HD Diesel Engines), et peut s'écrire sous la forme :

$$\log(NOx_{QS}) = a_0 + a_1 * COC + a_2 * m_{cyl} + a_3 * m_{O2}$$

Les coefficients $a_0$, $a_1$, $a_2$, $a_3$ sont obtenus à partir de données expérimentales et peuvent être calés en cours de fonctionnement au moyen des mesures d'un capteur, en l'occurrence un capteur d'oxydes d'azote NOx. Un des avantages de ce modèle est que ces coefficients varient peu d'un moteur à l'autre. Ce point est démontré dans l'article de Gartner précité.

Les particules en sortie du moteur sont la combinaison de deux phénomènes : la formation et la post-oxydation dans la chambre de combustion. Ces phénomènes sont au premier ordre influencés par la richesse, le régime, la quantité de carburant, et le taux de gaz brûlés. Ainsi, le modèle statique de particules en sortie du moteur peut s'écrire sous une équation de la forme :

$$Soot_{QS} = f(AF_{ratio}, Ne, Fuel, BGR)$$

La fonction f peut être déterminée par corrélation avec des données expérimentales et peut être calée en cours de fonctionnement au moyen des mesures d'un capteur, en l'occurrence un capteur de particules.

Des modèles similaires peuvent être construits pour les autres polluants (HC, CO).

Pour le mode de réalisation pour lequel on détermine l'impact des phénomènes transitoires, on peut mettre en oeuvre en outre les moyens décrits ci-dessous. Les phénomènes de dynamique de boucle d'air génèrent un écart sur les taux de BGR (fraction de gaz brûlés, liée à la recirculation des gaz d'échappement) et la richesse par rapport au point de fonctionnement stabilisé, ce qui a un impact fort sur les polluants, en particuliers les oxydes d'azote NOx, les hydrocarbures HC, le monoxyde de carbone CO et les particules. Les modèles d'impact du transitoire sont paramétrés en fonction de paramètres macroscopiques du moteur, en particulier des caractéristiques de la boucle d'air récupérées (atmosphérique/suralimentée, recirculation des gaz brûlés haute pression $EGR_{HP}$ / recirculation des gaz brûlés basse pression $EGR_{BP}$).

Ces modèles permettent d'estimer les fractions de gaz brulés et richesses dynamiques à partir des estimations quasi-statiques et de la variation du couple estimé :

$$BGR_{dyn} = f(BGR, Cme, dCme/dt)$$

$$AF_{ratio-dyn} = f(AF_{ratio}, Cme, dCme/dt)$$

Un coefficient de correction pour chaque polluant peut être calculé en fonction de ces grandeurs dynamiques :

$$Cor_{i-QS2TR} = f(BGR_{dyn}, BGR, AF_{ratio-dyn}, AF_{ratio})$$

Ces coefficients de correction permettent d'estimer les émissions polluantes en sortie moteur en prenant compte les phénomènes transitoires. Pour cela, les émissions de polluants en sortie du moteur peuvent s'écrire par une formule du type :

$$PSME_i = Cor_{i-QS2TR} * PSME_{i-QS}$$

Les signaux issus d'un capteur de dioxyde de carbone $CO_2$, d'oxyde de carbone CO, d'hydrocarbures, de oxydes d'azote NOx ou de particules (en particulier surtout un capteur d'oxydes d'azote NOx et un capteur de dioxyde de carbone $CO_2$) peuvent aider au recalage de la cartographie d'un système de recirculation des gaz brûlés EGR pouvant équipé le moteur.

Modèle du post-traitement

Le modèle du post-traitement relie les émissions de polluants en sortie du moteur (c'est-à-dire avant le système de post-traitement) aux émissions de polluants en sortie du système de post-traitement, de préférence au moyen d'au moins un paramètre macroscopique. Selon une mise en oeuvre de l'invention, pour construire le modèle du moteur on peut utiliser au moins un des paramètres macroscopiques suivants : la cylindrée, la norme d'homologation du véhicule, etc.

Le modèle de post-traitement peut comprendre des sous-modèles pour chaque technologie de dépollution, qui sont associés en fonction de l'architecture du système de dépollution du véhicule. Ces sous-modèles peuvent être paramétrés en fonction de paramètres macroscopiques du véhicule tels que la norme d'homologation, la cylindrée, etc. Par exemple, les différentes technologies de dépollution peuvent être :

- TWC, de l'anglais « Three-way catalytic converters » signifiant catalyseur trois voies,
- GPF (pour moteur essence), de l'anglais « gasoline particle filter » signifiant filtre à particules pour essence,
- DOC (pour moteur diesel), de l'anglais « Diesel oxidation catalyst », signifiant catalyseur à oxydation pour Diesel,
- DPF (pour moteur diesel), de l'anglais « Diesel particle filter », signifiant filtre à particules pour Diesel,
- LNT (pour moteur Diesel), de l'anglais « lean Nox trap », signifiant piège à Nox,
- SCR (pour moteur Diesel), de l'anglais « Selective catalytic reduction », signifiant réduction catalytique sélective.

Le modèle de post-traitement permet d'estimer les émissions de polluants en sortie du système de post-traitement à partir des estimations de température, débits, et émissions polluantes en sortie du moteur. Le modèle de post-traitement peut être construit en discrétisant le système de post-traitement en plusieurs tranches (ou couches), et par association de l'efficacité $Conv_{i,j}$ de chaque tranche discrétisée. Selon un exemple, le modèle de post-traitement peut s'écrire :

$$PSEE_i = \prod_{j=1}^{Nb\,pain} Conv_{i,j}(T_{\acute{e}ch}, Q_{\acute{e}ch}) * PSME_i$$

L'efficacité des tranches du système de post-traitement peut être déterminée à partir de cartographies du constructeur et peut être calée en cours de fonctionnement au moyen des mesures d'un capteur.

Etapes de détermination des émissions de polluant au moyen du système embarqué de mesure

Selon un mode de réalisation non limitatif, le système embarqué de mesure peut mettre en oeuvre les étapes suivantes pour déterminer les émissions polluantes

1) Mesure du capteur et transmission au téléphone intelligent
2) Mesure de la géolocalisation (facultatif) et transmission au nuage informatique
3) (étape facultative) Prétraitement des mesures
4) Détermination du couple et du régime moteur

5) Détermination des émissions de polluants en sortie du moteur
6) Détermination des émissions de polluants du véhicule
7) (étape facultative) Stockage des données

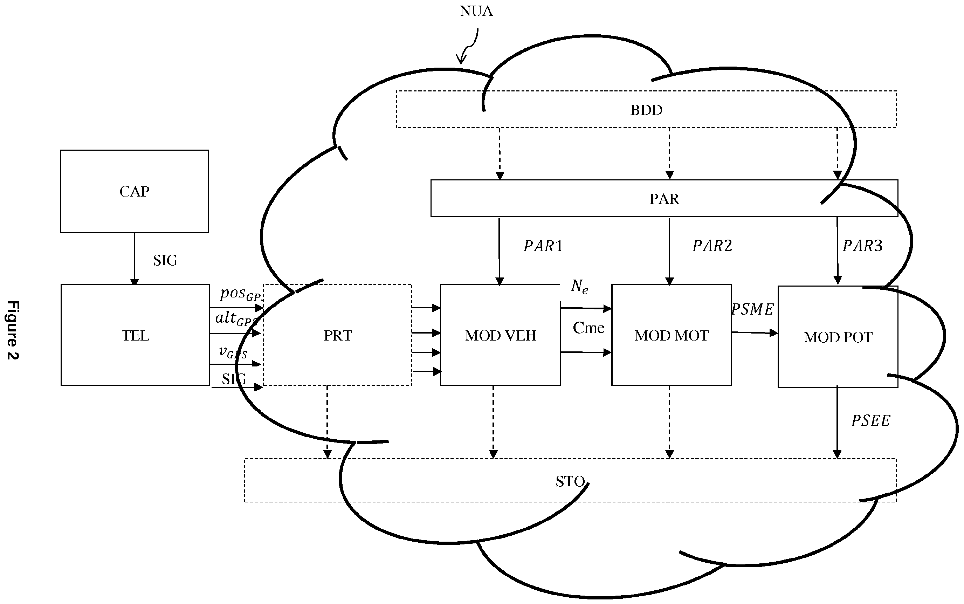

La figure 2 illustre, de manière schématique et non limitative, les étapes du procédé d'utilisation du système embarqué de mesure selon un mode de réalisation de l'invention. Sur cette figure, les traits pointillés indiquent des éléments facultatifs du procédé.

Préalablement aux étapes du procédé, les différents modèles (modèle du véhicule MOD VEH, modèle du moteur MOD MOT et modèle du post-traitement MOD POT) sont construits. Ces modèles sont construits de préférence à partir de paramètres macroscopiques PAR. De manière facultative, les paramètres macroscopiques PAR peuvent être obtenus depuis une base de données BDD, qui répertorie les différents véhicules en circulation. Par exemple, les paramètres macroscopiques PAR peuvent être obtenus en indiquant le numéro d'immatriculation du véhicule, la base de données BDD associant le numéro de plaque à la conception du véhicule (marque, modèle, motorisation...), et comprenant les paramètres macroscopiques du véhicule. La base de données BDD peut être stockée dans le nuage informatique NUA du système informatique.

Une première série de paramètres macroscopiques PAR1 peut être utilisée pour la construction du modèle du véhicule MOD VEH. Cette première série de paramètres macroscopiques PAR1 peut comprendre les paramètres suivants : la masse du véhicule, la puissance maximale et le régime du moteur associé, la vitesse maximale, le type de transmission (liste non limitative).

Une deuxième série de paramètres macroscopiques PAR2 peut être utilisée pour la construction du modèle du moteur MOD MOT. Cette deuxième série de paramètres macroscopiques PAR2 peut comprendre les paramètres suivants : la cylindrée, le type de motorisation, le couple et la puissance maximums, l'architecture de la boucle d'air, la norme d'homologation du véhicule (liste non limitative).

Une troisième série de paramètres macroscopiques PAR3 peut être utilisée pour la construction du modèle du post-traitement MOD POT. Cette troisième série de paramètres macroscopiques PAR3 peut comprendre les paramètres suivants : la cylindrée, la norme d'homologation du véhicule (liste non limitative).

Ces trois modèles peuvent être construits selon l'une des variantes de réalisation décrites ci-dessus.

La première étape consiste en une étape de mesure par le capteur CAP en sortie du système de post-traitement du véhicule, puis une transmission de cette mesure (signal SIG) au téléphone intelligent TEL. Cette transmission au téléphone intelligent TEL peut être mise en oeuvre au moyen d'une connexion sans fil.

La deuxième étape peut consister en une étape de mesure MES de géolocalisation. Lors de cette étape, on peut mesurer la position $pos_{GPS}$, et/ou l'altitude $alt_{GPS}$ et/ou la vitesse $v_{GPS}$ du véhicule. La prise en compte de l'altitude $alt_{GPS}$ permet notamment de prendre en compte la pente de la route. De préférence, on réalise les trois mesures, de manière à avoir l'information la plus précise possible concernant la géolocalisation du véhicule, car on peut prendre alors en compte le style de conduite, et l'accélération du véhicule. Cette mesure peut être réalisée à l'aide d'un système de géolocalisation, par exemple de type GPS (pour « global positioning system » signifiant géo-positionnement par satellite) ou de type Galileo, ou au moyen d'un téléphone intelligent (« smartphone »), etc. Dans le cas d'un téléphone intelligent, celui-ci peut être équipé d'un système de géolocalisation, alternativement les mesures peuvent être mises en oeuvre par d'autres moyens, notamment par triangulation.

De plus, la deuxième étape comprend la transmission au nuage informatique NUA de la mesure issue du capteur, et le cas échéant des mesures de géolocalisation.

La troisième étape, qui est une étape facultative, est une étape de prétraitement PRT des signaux de mesure. Cette étape permet d'améliorer la qualité des signaux mesurés avant de les utiliser. Cette étape peut notamment être intéressante, si les mesures sont réalisées à partir d'un téléphone intelligent, car les mesures issues d'un tel dispositif peuvent être quelque peu imprécises. Ce prétraitement peut être variable, car il est dépendant de la qualité des données d'entrée. Selon un mode de réalisation de l'invention, le prétraitement PRT peut comprendre un sur-échantillonnage des signaux, puis un filtrage. En sortie de cette étape, on retrouve donc des signaux relatifs à la position $pos_{GPS}$, et/ou l'altitude $alt_{GPS}$ et/ou la vitesse $v_{GPS}$ du véhicule, ces signaux ayant été prétraités.

La quatrième étape concerne la détermination du couple et du régime moteur. Cette étape est mise en oeuvre au moyen du modèle du véhicule MOD VEH, qui détermine le couple Cme et le régime Ne du moteur, en fonction des données de géolocalisation : la position $pos_{GPS}$, et/ou l'altitude $alt_{GPS}$ et/ou la vitesse $v_{GPS}$ du véhicule. De plus, lors de cette étape, le modèle du véhicule MOD VEH peut être calé à l'aide du signal SIG du capteur CAP, pour améliorer la précision du modèle de véhicule MOD VEH.

La cinquième étape concerne la détermination des émissions polluantes en sortie du moteur, cette étape est mise en oeuvre au moyen du modèle du moteur MOD MOT, qui détermine les émissions de polluants en sortie du moteur PSME, en fonction du couple Cme et du régime Ne du moteur. De plus, lors de cette étape, le modèle du moteur MOD MOT peut être calé à l'aide du signal SIG du capteur CAP, pour améliorer la précision du modèle du moteur MOD MOT.

La sixième étape concerne la détermination des émissions de polluants du véhicule, c'est-à-dire en sortie du système de post-traitement. La détermination des émissions de polluants peut être réalisée à chaque instant, par exemple à une fréquence de 1Hz. De plus, il est également possible de déterminer le cumul des émissions de polluants sur un trajet réalisé. Cette étape est mise en oeuvre au moyen du modèle de post-traitement MOD POT, qui détermine les émissions de polluants en sortie du système de post-traitement PSEE, en fonction des émissions de polluants en sortie du moteur PSME. De plus, lors de cette étape, le modèle du système de post-traitement MOD POT peut être calé à l'aide du signal SIG du capteur CAP, pour améliorer la précision du modèle de post-traitement MOD POT.

La septième étape, facultative, concerne le stockage des données. Une fois que les émissions de polluants du véhicule PSEE sont déterminées, celles-ci peuvent être stockées STO (enregistrées), en particulier dans une base de données (différente de la base de données qui comportent les paramètres macroscopiques). Ce stockage STO peut concerner uniquement les émissions de polluants du véhicule PSEE, mais peut aussi concerner les données déterminées après chaque étape du procédé : les mesures prétraitées et/ou le couple Cme et le régime Ne du moteur et/ou les émissions de polluants en sortie du moteur PSME. Ces informations permettent un suivi des usages réels et des émissions associées avec une bonne résolution spatiale et temporelle. Ces informations peuvent par exemple permettre d'évaluer la pertinence environnementale des infrastructures routières à l'échelle d'une rue, identifier des pics d'émissions localisés, identifier l'impact du style de la conduite sur les émissions, etc. Le stockage STO peut être contenu dans le nuage informatique NUA.

Cette base de données peut associer les émissions instantanées aux données cartographiques pour constituer une carte des émissions polluantes réelles. Il est donc possible de tirer des conclusions à l'échelle d'une portion de route, d'un trajet complet ou même d'une zone géographique selon les besoins. Lors de cette étape, il est également possible d'afficher les émissions de polluants en sortie du véhicule PSEE, par exemple sur un écran d'un système de géolocalisation (GPS, Galiléo), d'un téléphone intelligent, sur le tableau de bord du véhicule, sur un site internet, etc. Ainsi, il est possible d'informer l'utilisateur ou tout autre personne (par exemple un gestionnaire de flotte de véhicules, un responsable d'infrastructure routière...) de la pollution émise sur un trajet ou sur une route.

### Revendications

1. Système embarqué de mesure des émissions polluantes d'un véhicule, ledit véhicule comportant un moteur à combustion interne et un système de post-traitement des gaz d'échappement dudit moteur, ledit système embarqué comportant au moins un capteur (CAP) placé en aval dudit système de post-traitement dudit véhicule et un système informatique (SIN) de détermination des émissions polluantes dudit véhicule au moyen de la mise en oeuvre d'un modèle du véhicule (MOD VEH), d'un modèle de la motorisation (MOD MOT) dudit véhicule et d'un modèle de post-traitement (MOD POT) dudit véhicule, ledit modèle de véhicule (MOT VEH) reliant ladite position et/ou l'altitude et/ou la vitesse dudit véhicule au couple et au régime dudit moteur, ledit modèle de motorisation (MOD MOT) reliant ledit couple et ledit régime dudit moteur aux émissions de polluants en sortie dudit moteur, et ledit modèle de post-traitement (MOD POT) reliant lesdites émissions de polluants en sortie dudit moteur aux émissions de polluants en sortie dudit système de post-traitement, **caractérisé en ce que** lesdits modèles (MOD VEH, MOD MOT, MOD POT) dudit système informatique (SIN) sont configurés pour prendre en compte un signal issu dudit au moins un capteur (CAP) afin de déterminer les émissions polluantes dudit véhicule.

2. Système embarqué de mesure selon la revendication 1, dans lequel ledit système informatique (SIN) comprend un téléphone intelligent (TEL).

3. Système embarqué de mesure selon la revendication 2, dans lequel ledit au moins capteur (CAP) communique avec ledit téléphone intelligent (TEL) au moyen d'une connexion sans fil.

4. Système embarqué de mesure selon l'une des revendications 2 ou 3, dans lequel lesdits modèles (MOD VEH, MOD MOT, MOD POT) dudit système informatique (SIN) sont enregistrés dans un nuage informatique (NUA), ledit téléphone intelligent (TEL) étant configuré pour échanger avec ledit nuage informatique (NUA).

5. Système embarqué de mesure selon l'une des revendications 2 à 4, dans lequel ledit téléphone intelligent (TEL) mesure la position et/ou l'altitude et/ou la vitesse dudit véhicule pour déterminer les émissions polluantes au moyen desdits modèles (MOD VEH, MOD MOT, MOD POT).

6. Système embarqué de mesure selon l'une des revendications précédentes, dans lequel ledit système informatique (SIN) comporte des moyens d'enregistrement (STO) des émissions polluantes déterminées au moyen desdits modèles.

**7.** Système embarqué de mesure selon l'une des revendications précédentes, dans lequel ledit système embarqué de mesure comprend en outre un capteur branché sur la prise de diagnostic dudit véhicule.

**8.** Système embarqué de mesure selon l'une des revendications précédentes, dans lequel ledit au moins un capteur (CAP) est choisi parmi un capteur d'oxydes d'azote NOx, un capteur de monoxyde de carbone CO ou de dioxyde de carbone $CO_2$, un capteur de dioxygène $O_2$, un capteur d'hydrocarbures non brûlés, un capteur de particules.

**9.** Système embarqué de mesure selon la revendication 8, dans lequel ledit capteur d'oxyde d'azote NOx est choisi parmi capteur électrochimique, un capteur ultraviolet UV, un capteur infrarouge IR, un détecteur à chimie luminescente CLD.

**10.** Système embarqué de mesure selon l'une des revendications 8 à 9, dans lequel ledit capteur de monoxyde de carbone CO ou de dioxyde de carbone $CO_2$ est choisi parmi un capteur ultraviolet UV ou un capteur infrarouge IR.

**11.** Système embarqué de mesure selon l'une des revendications 8 à 10, dans lequel ledit capteur de dioxygène $O_2$ est choisi parmi un capteur électrochimique, une mesure par diode LASER, un détecteur paramagnétique, un détecteur magnéto-pneumatique.

**12.** Système embarqué de mesure selon l'une des revendications 8 à 11, dans lequel ledit capteur de particules est choisi parmi un comptage par décharge CORONA, un opacimètre optique, un capteur de particules par accumulation, un comptage par vapeur de butanol, un comptage par irradiation des particules, un détecteur de fumée optique ou ionique, un détecteur d'atténuation beta BAM, un compteur de particules LASER.

**13.** Système embarqué de mesure selon l'une des revendications 8 à 12, dans lequel ledit système embarqué comporte un capteur d'oxydes d'azote NOx, et un capteur de dioxyde de carbone $CO_2$.

**14.** Système embarqué de mesure selon l'une des revendications précédentes, dans lequel lesdits modèles (MOD VEH, MOD MOT, MOD POT) sont configurés pour prendre en compte le signal issu dudit au moins un capteur (CAP) par un calage desdits modèles (MOD VEH, MOD MOT, MOD POT) par comparaison des émissions polluantes déterminées par lesdits modèles (MOD VEH, MOD MOT, MOD POT) avec les émissions polluantes mesurées par ledit au moins un capteur (CAP).

**15.** Système embarqué de mesure selon la revendication 14, dans lequel le calage est mis en oeuvre au moyen d'une méthode de résolution par les moindres carrés et/ou par un filtre de Kalman.

**16.** Système embarqué de mesure selon l'une des revendications précédentes, dans lequel ledit système informatique (SIN) est configuré en outre pour déterminer un paramètre descriptif du trajet dudit véhicule.

**17.** Système embarqué de mesure selon l'une des revendications précédentes, dans lequel lesdits modèles (MOD VEH, MOD MOT, MOD POT) dépendent en outre d'au moins un paramètre macroscopique (PAR) dudit véhicule.

**18.** Système embarqué de mesure selon la revendication 17, dans lequel ledit système comporte des moyens d'acquisition dudit au moins un paramètre macroscopique (PAR) depuis une base de données (BDD) et/ou au moyen d'une interface avec un utilisateur.

Figure 1

Figure 2

Europäisches
Patentamt

European
Patent Office

Office européen
des brevets

# RAPPORT DE RECHERCHE EUROPEENNE

Numéro de la demande

EP 19 15 6700

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X<br>Y | WO 2017/174239 A1 (IFP ENERGIES NOW [FR])<br>12 octobre 2017 (2017-10-12)<br>* page 5, ligne 31 - page 17, ligne 32;<br>figures *<br>----- | 1-13,<br>16-18<br>14,15 | INV.<br>F02D41/14<br>F02D41/26<br>G01N27/00<br>G01M15/10 |
| Y<br><br><br>A | WO 02/08582 A1 (BOSCH GMBH ROBERT [DE];<br>SCHNAIBEL EBERHARD [DE]; WINKLER KLAUS<br>[DE]) 31 janvier 2002 (2002-01-31)<br>* page 12 - page 13; figures *<br>----- | 14<br><br><br>8,9,13 | G01N21/00<br><br>ADD.<br>F02D41/00<br>F02D41/28 |
| Y<br>A | DE 10 2010 046491 A1 (IAV GMBH [DE])<br>29 mars 2012 (2012-03-29)<br>* alinéa [0021] - alinéa [0031] *<br>----- | 14,15<br>8-13 | |
| Y<br>A | US 2005/267669 A1 (WANG WEI [US] ET AL)<br>1 décembre 2005 (2005-12-01)<br>* alinéa [0016] - alinéa [0050]; figure 1<br>*<br>----- | 14,15<br>8-11,13 | |
| A | DE 10 2013 206308 A1 (BOSCH GMBH ROBERT<br>[DE]) 16 octobre 2014 (2014-10-16)<br>* alinéa [0005] - alinéa [0022] *<br>----- | 1-18 | DOMAINES TECHNIQUES RECHERCHES (IPC)<br><br>F02D |

Le présent rapport a été établi pour toutes les revendications

| Lieu de la recherche | Date d'achèvement de la recherche | Examinateur |
|---|---|---|
| Munich | 21 mars 2019 | Aign, Torsten |

CATEGORIE DES DOCUMENTS CITES

X : particulièrement pertinent à lui seul
Y : particulièrement pertinent en combinaison avec un
autre document de la même catégorie
A : arrière-plan technologique
O : divulgation non-écrite
P : document intercalaire

T : théorie ou principe à la base de l'invention
E : document de brevet antérieur, mais publié à la
date de dépôt ou après cette date
D : cité dans la demande
L : cité pour d'autres raisons

                                          
& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 19 15 6700

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

21-03-2019

| Document brevet cité au rapport de recherche | | Date de publication | Membre(s) de la famille de brevet(s) | | Date de publication |
|---|---|---|---|---|---|
| WO 2017174239 | A1 | 12-10-2017 | CN | 108884770 A | 23-11-2018 |
| | | | EP | 3440330 A1 | 13-02-2019 |
| | | | FR | 3049653 A1 | 06-10-2017 |
| | | | WO | 2017174239 A1 | 12-10-2017 |
| WO 0208582 | A1 | 31-01-2002 | DE | 10036453 A1 | 14-02-2002 |
| | | | EP | 1307639 A1 | 07-05-2003 |
| | | | JP | 5220258 B2 | 26-06-2013 |
| | | | JP | 2004504539 A | 12-02-2004 |
| | | | US | 2003163987 A1 | 04-09-2003 |
| | | | WO | 0208582 A1 | 31-01-2002 |
| DE 102010046491 | A1 | 29-03-2012 | AUCUN | | |
| US 2005267669 | A1 | 01-12-2005 | AUCUN | | |
| DE 102013206308 | A1 | 16-10-2014 | AUCUN | | |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 3049653
- WO 17174239 A
- FR 2984557
- US 20130158967 A